(19) Europäisches Patentamt
European Patent Office
Office européen des brevets

(11) Publication number: **0 117 412 A1**

(12) # EUROPEAN PATENT APPLICATION

(21) Application number: **84100497.1**

(22) Date of filing: **18.01.84**

(51) Int. Cl.³: **C 07 C 59/66**, C 07 C 69/736, C 07 C 101/28, C 07 C 101/453, C 07 C 103/58, C 07 C 101/34, C 07 C 101/44, C 07 C 143/80, C 07 C 149/32, C 07 D 213/62, C 07 D 239/32

(30) Priority: **18.01.83 JP 6110/83**
**07.02.83 JP 18600/83**
**10.02.83 JP 21281/83**
**25.02.83 JP 29390/83**
**08.07.83 JP 123470/83**
**08.07.83 JP 123471/83**
**14.09.83 JP 168442/83**

(43) Date of publication of application: **05.09.84**
**Bulletin 84/36**

(84) Designated Contracting States: **CH DE FR GB LI NL**

(71) Applicant: **DAICEL CHEMICAL INDUSTRIES, LTD.,**
**No. 1, Teppo-cho, Sakai-shi Osaka 590 (JP)**

(72) Inventor: **Yagihara, Hiroshi, 1177, Matogata Matogata-cho, Himegi Hyogo (JP)**
Inventor: **Hirako, Yoshiyuki, 500, Kamiyobe Yobe-ku, Himeji Hyogo (JP)**
Inventor: **Morishima, Yasuo, 6-11, 3-chome, Tsutsujigaoka, Tarumi-ku Kobe (JP)**
Inventor: **Masamoto, Kazuhisa, 500, Kamiyobe Yobe-ku, Himeji Hyogo (JP)**

(74) Representative: **Hansen, Bernd, Dr.rer.nat. et al,**
**Hoffmann, Eitle & Partner Patentanwälte**
**Arabellastrasse 4, D-8000 München 81 (DE)**

(54) **Propene derivatives and their use as plant growth inhibitors.**

(57) A propene derivative represented by general formula:

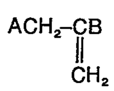
(I)

wherein A and B are as defined hereinbefore. The propene derivative of the invention is useful as a plant growth inhibitor.

EP 0 117 412 A1

ACTORUM AG

Propene derivatives and their use as plant growth inhibitors

The present invention relates to novel propene derivatives having specific substituents and, more particularly, to α-methylenepropionic acids having specific substituents at the β-position thereof and functional derivatives thereof, such as esters, nitriles, etc., as well as 4-chromanone derivatives. The compounds are useful as plant growth inhibitors.

β-Phenoxy-α-methylenepropionic acid has been reported as an intermediate for the synthesis of 3-methylcoumarin. Further β-phenylthio-α-methylenepropionic acid has been reported as an intermediate for the synthesis of thiochroman-3-carboxylic acid (Swaminathan et al, Synthesis Communications, Sept. 1975, page 599, and ibid, June 1976, page 409). With respect to nucleus-substituted derivatives of these compounds, some are merely mentioned in the articles described above. In these articles, nothing is mentioned with respect to plants. Further, no ester of the aforesaid compounds is mentioned.

On the other hand, as nitriles of α-methylenepropionic acid having a substituent at the β-position thereof,

that is, α-substituted acrylonitriles having a substituent of the $ACH_2$ type at the α-position thereof, ethoxymethyl, propoxymethyl, cyclohexyloxymethyl, etc. are reported as α-substituents by Movsum-Zada E.M., et al, Azerb. Khim. Zh., (5), 70 - 73 (1976), and methylthiomethyl is reported as the α-substituent by La Combe, E.M., et al, JACS, 83, 3457-3461 (1961). Further, examples of a dimethylaminomethyl group and a dinonylaminomethyl group are disclosed in Gner Nathaniel et al, European Patent Application 31584, and a benzylaminomethyl group is mentioned in Congnacq Jean C. et al, Canadian Patent 960,666; however, no effect on plants is mentioned therein.

In Japanese Patent Publication No. 1972/77, certain α-substituted acrylic acid derivatives are mentioned as plant growth regulators; however, these compounds have a structure of the thiol ester of chain aliphatic thioether type.

An object of the present invention is to provide novel, useful compounds and more particularly, to provide useful compounds having an action on plants. This object was attained by synthesizing a series of propene derivatives represented by general formula:

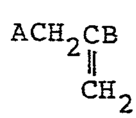

(I)

wherein A and B will be later defined, and confirming the effective actions of these compounds on plants.

The compounds of the present invention are represented by general formula (I) described above wherein:

A represents an atomic group bonded to the saturated carbon atom of the propene group via an oxygen atom, a sulfur atom or a nitrogen atom;

B represents an atomic group which is selected from an alkoxy($C_1$-$C_4$)carbonyl group, a cyano group, a substituted aminocarbonyl group represented by the formula, $-CON\begin{smallmatrix} R_1 \\ R_2 \end{smallmatrix}$ (wherein $R^1$ and $R^2$ represent a group selected from the group consisting of a lower alkyl group having 1 to 4 carbon atoms, phenyl, benzyl and $-C_6H_5SO_2NH_2$), an isopropylideneaminoxycarbonyl group, a phenyloxycarbonyl group, a phenylthiocarbonyl group, a carboxyl group and a carbonyl group which is bound to a benzene nucleus in A to form a 4-chromanone skeleton; and;

A above being the following, depending upon type of Compound (I):

(1) in case that Compound (I) is an alkyl ester or a nitrile:

phenoxy, non-cyclic group-substituted phenoxy (having as substituent(s) one or two selected from the group

consisting of methyl, isopropyl, trifluoromethyl, methoxy, methylthio, fluoro, chloro and cyano), phenoxyphenoxy, substituted phenoxyphenoxy (having as substituent(s) one or two selected from the group consisting of fluoro, nitro, chloro, methyl, methoxy and trifluoromethyl), heterocyclic group-substituted oxyphenoxy (having as the substituted oxy group 2-benzothiazolyloxy, 2-pyridyloxy, substituted (chloro- or nitro-)-2-pyridyloxy, 2-pyrimidyloxy, 2-quinolyloxy, 4-methyl-2-quinolyloxy, 3-quinolyloxy or 6-chloro-2-quinoxalyloxy), 2-benzothiazolylthiophenoxy, benzyloxyphenoxy, cyclic aminophenoxy (having as the cyclic amino group anilino, 2-naphthylamino or N-methyl-N-(2-benzothiazolyl)amino), 4-phenylphenoxy, 4-phenylethenylphenoxy, 5-tetralinoxy, 2-fluorenyloxy, 5-quinolyloxy, 4-methyl-7-coumarinyloxy, diethylaminoxy, phenylthio, non-cyclic group-substituted phenylthio (having as substituent(s) one to three groups selected from the group consisting of methyl, methoxy, chloro and amino), benzylthio, 4-chlorobenzylthio, cyclohexylthio, 1-naphthylthio, 2-naphthylthio, 2-pyridylthio, 2-pyrimidylthio, 2-benzothiazolylthio, anilino, substituted anilino (having as the substituent(s) one or two groups selected from the group consisting of methyl and chloro), N-methylanilino, pyridylamino and α-methylbenzylamino;

(2) in case that Compound (I) is an amide, an oxime ester, a phenyl ester or a thiophenyl ester:

phenoxy;

(3) in case that Compound (I) is a carboxylic acid:

4-fluorophenoxy, 4-methylthiophenoxy, 4-phenylphenoxy, 4-α-naphthylaminophenoxy or 5-tetralinoxy;

(4) in case that Compound (I) is a 4-chromanone compound:

a substituted phenoxy (the substituent(s) being one or two of members selected from the group consisting of methyl, methoxy, chloro and fluoro).

The compounds of the present invention are propene derivatives represented by general formula (I):

$$\begin{array}{l} ACH_2CB \\ \quad\;\;\; \| \\ \quad\;\;\; CH_2 \end{array} \qquad (I)$$

In general formula (I), A is an atomic group bonded to the saturated carbon atom of the propene via an oxygen atom, a sulfur atom or a nitrogen atom. When A is represented as D-T-, T is O, S, NH or $NCH_3$ and D often includes a cyclic group such as a benzene nucleus, etc. B is a carboxyl group, an alkoxy($C_1$-$C_4$)carbonyl group or a cyano group or functional groups equivalent to these groups. The compounds of the present invention belong to compounds of types II to VI in Table 1 depending upon the kind of B classified in Table 2.

Table 3

| Type | Atomic Group A |
|---|---|
| II or III | $C_6H_5O$, $C_6H_5OC_6H_4O$, |

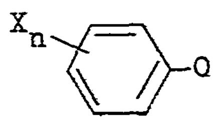 [n is 1 or 2, X is $CH_3$, $i-C_3H_7$, $F_3C$, $CH_3O$, $CH_3S$, F, Cl or CN] ,

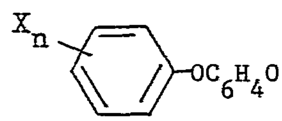 [n is 1 or 2, X is F, $NO_2$, Cl, $CH_3$, $CH_3O$ or $F_3C$] ,

$YOC_6H_4O$ [YO is 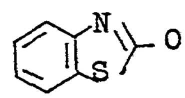 ,

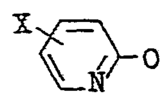 (X is H, Cl or $NO_2$),

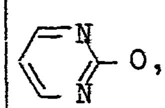 , 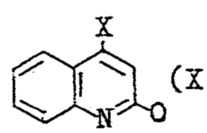 (X is H or $CH_3$),

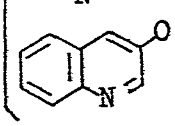 , 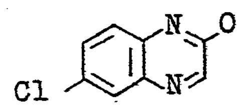 ],

$C_6H_5CH_2OC_6H_4O$, 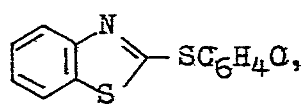 ,

$Y-NR-C_6H_4O$ [Y-NR- is $C_6H_5NH-$ , 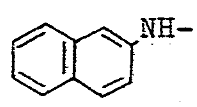

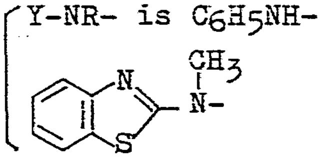 ],

$C_6H_5C_6H_4O$, $C_6H_5CH=CHC_6H_4O$,

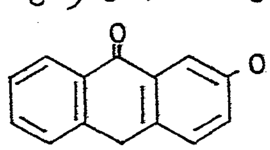 , 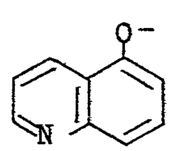 , 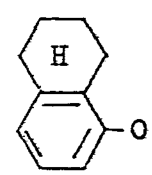 ,

Table 3 (Cont'd)

| Type | Atomic Group A |
|---|---|
| | 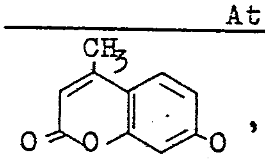 , $(C_2H_5)_2NO-$ |
| | $C_6H_5S$,<br>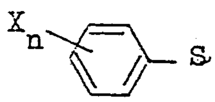 (n is 1, 2 or 3; X is $CH_3$, $CH_3O$, Cl, $NH_2$)<br>$C_6H_5CH_2S$, $Cl-C_6H_4CH_2S$, 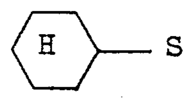 ,<br>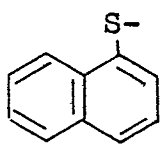 , 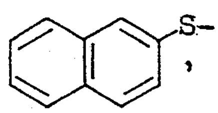 , 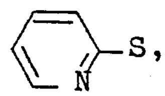 , 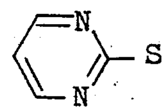 ,<br>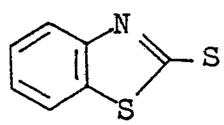 |
| | $C_6H_5NH$, 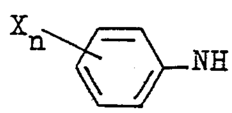 (n is 1 or 2; X is $CH_3$, Cl) ,<br>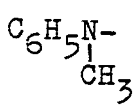 , 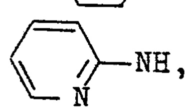 , 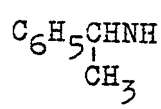 |
| IV | $C_6H_5O$ |
| V | $FC_6H_4O-$, $CH_3SC_6H_4O-$, $C_6H_5-C_6H_4O$,<br>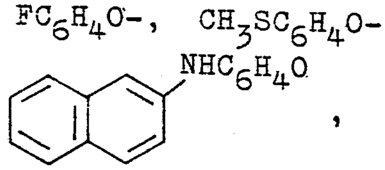 , 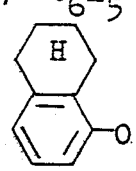 |

Table 3 (cont'd)

| Type | Atomic Group A | |
|---|---|---|
| VI | 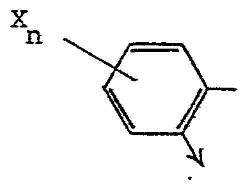 | n is 1 or 2<br>X is $CH_3$, $CH_3O$, Cl, F<br>bound to the carbonyl of B<br>at the arrow position |

Representative examples of the compounds of the present invention which possess combinations of specific A and B moieties described above are shown in Tables 4 to 10. These tables also show NMR analysis data which are useful for the identification of the respective compounds of the present invention.

Table 4

Compound II (B=COOCH$_3$ in formula (I))

| Compound No. | A(=-T-D) | NMR (CDCℓ$_3$) δ value: -CO$_2$CH$_3$ | -T-CH$_2$- | =CH$_2$ | T-portion | IR (cm$^{-1}$) $\nu_{c=o}$ | $\nu_{c=c}$ | Property (melting point) |
|---|---|---|---|---|---|---|---|---|
| 4-1 | 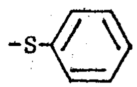 | 3.76(S,3H) | 3.76(S,2H) | 5.52(m,1H) 6.12 " | 7.10~7.60(m,5H) | 1725 | 1633 | light yellow oily product |
| 4-2 | 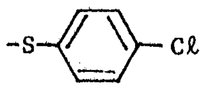 | 3.75 " | 3.70(S*,2H) | 5.48(S*,1H) 6.12 " | 7.00~7.30( " ) | 1720 | 1628 | colorless oily product |
| 4-3 | 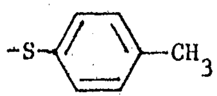 | 3.75 " | 3.70(S,2H) | 5.47(S,1H) 6.13 " | 7.07(d,2H) 7.26(d,2H) -CH$_3$ 2.30 (S,3H) | 1719 | 1620 | " |
| 4-4 | 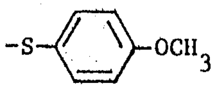 | 3.75 " | 3.60 " | 5.30 " | 6.77(d,2H) 7.23(d,2H) -OCH$_3$ 3.60(S,3H) | 1718 | 1620 | light yellow oily product |
| 4-5 | 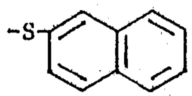 | 3.77 " | 3.87 " | 5.58 " 6.17 " | 7.20~7.90(m,5H) | 1710 | 1618 | " |
| 4-6 | 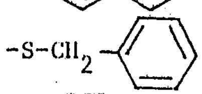 | 3.74 " | 3.64(S*,2H) | 5.57(m,1H) 6.18 " | 7.15~7.35( " ) -CH$_2$-Ph 3.27(S,2H) | 1722 | 1628 | colorless oily product |
| 4-7 | 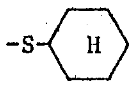 | 3.76 " | 3.42 " | 5.68 " 6.17 " | 1.00~2.90(m,11H) | 1730 | 1635 | " |
| 4-8 | 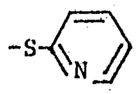 | 3.87 " | 4.26(S,2H) | 6.10(S,1H) 6.40 " | 7.00~7.80(m,3H) 8.50~8.70(m,1H) | 1722 | 1632 | light yellow oily product |
| 4-9 | 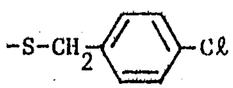 | 3.75 " | 3.61( " ) | 5.55(S,1H) 6.17 | 6.90~7.40(m,4H) 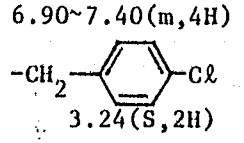 3.24(S,2H) | 1715 | 1620 | colorless oily product |

*NMR (CDCℓ$_3$-DMSO-d$_6$)

**Table 4** (cont'd)

Compound II (B=$COOCH_3$ in formula (I))

| Compound No. | A(=-T-D) | NMR ($CDCl_3$) δ value: $-CO_2CH_3$ | $-T-CH_2-$ | $=CH_2$ | T-portion | IR ($cm^{-1}$) $\nu_{c=o}$ | $\nu_{c=c}$ | Property (melting point) |
|---|---|---|---|---|---|---|---|---|
| 4-10 | 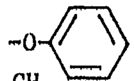 | 3.83 | 4.82(S*,2H) | 6.09(m,1H)<br>6.48 | 6.95~7.60(m,5H) | 1720 | 1645 | colorless oily product |
| 4-11 | 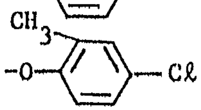 | 3.78(S,3H) | 4.70( " ) | 6.00 "<br>6.40 " | 6.50~7.30(m,3H)<br>$-CH_3$ 2.20(S,3H) | 1722 | 1635 | white (57~59.5PE) |
| 4-12 | 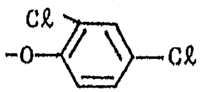 | 3.82 " | 4.81( " ) | 6.15 "<br>6.47 " | 6.70~7.55(m,3H) | 1720 | 1630 | white (60~61.5) |
| 4-13* | 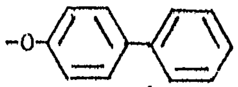 | 3.85 " | 4.84( " ) | 6.10 "<br>6.48 " | 6.90~7.70(m,9H) | 1720 | 1632 | white (75~77°) |
| 4-14 | 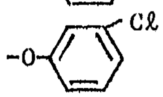 | 3.78 " | 4.72( " ) | 5.96 "<br>6.38 " | 6.70~7.30(m,4H) | 1710 | 1625 | light yellow oily product |
| 4-15 | 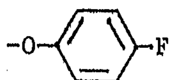 | 3.83 " | 4.75( " ) | 6.05 "<br>6.46 " | 6.70~7.15(m,4H) | 1715 | 1630 | colorless oily product |
| 4-16 | 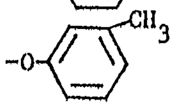 | 3.76 " | 4.72( " ) | 5.96 "<br>6.36 " | $-CH_3$ 2.29(S,3H) | 1710 | 1630 | " |
| 4-17 | 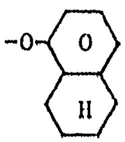 | 3.77(bs 1H) | 4.72(S,2H) | 6.03(S,1H)<br>6.38 | 1.60~2.00(m,4H)<br>2.50~7.20(m,4H)<br>6.50~7.20(m,3H) | 1715 | 1635 | light yellow oily product |
| 4-18 | 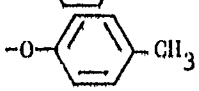 | 3.76 " | 4.69( " ) | 5.97 "<br>6.35 " | 6.60~7.20(m,4H) | 1714 | 1635 | " |

*NMR($CDCl_3$-$DMSO-d_6$)

Table 4 (cont'd)

Compound II (B=COOCH$_3$ in formula (I))

| Compound No. | A(=-T-D) | NMR (CDCl$_3$) δ value: -CO$_2$CH$_3$ | -T-CH$_2$- | =CH$_2$ | T-portion | IR (cm$^{-1}$): $\nu_{c=o}$ | $\nu_{c=c}$ | Property (melting point) |
|---|---|---|---|---|---|---|---|---|
| 4-19 | 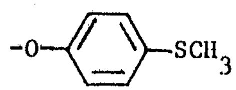 | 3.78(S,3H) | 4.72( " ) | 5.98(S,1H)<br>6.39 " | 6.70~7.40(m,4H)<br>-SCH$_3$ 2.42(S,3H) | 1720 | 1635 | light yellow oily product |
| 4-20 | 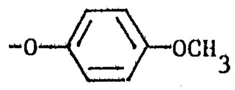 | 3.72(S,2H) | 4.62( " ) | 5.90 "<br>6.28 " | 6.70~7.00(m,4H)<br>-CH$_3$O- 3.68(S,3H) | 1720 | 1635 | " |
| 4-21 | 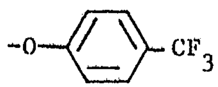 | 3.80(S,3H) | 4.77( " ) | 5.98 "<br>6.42 " | 6.80~7.70(m,4H) | 1715 | 1635 | colorless oily product |
| 4-22 | 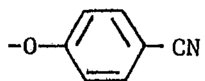 | 3.78 " | 4.78( " ) | 5.97 "<br>6.40 " | 6.80~7.70(m,4H) | 1715 | 1630 | white(55~58) cyclohex |
| 4-23 | 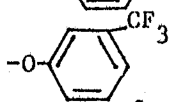 | 3.77 " | 4.76( " ) | 6.00 "<br>6.40 " | 6.90~7.50(m,4H) | 1715 | 1632 | colorless oily product |
| 4-24 | 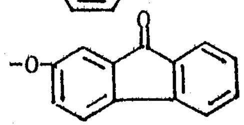 | 3.80 " | 4.77( " ) | 5.97 "<br>6.39 " | 6.70~7.60(m,7H) | 1710<br>1700 | 1630 | yellow (90~92.5) |
| 4-25 | 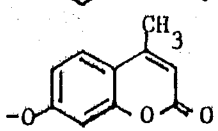 | 3.83 " | 4.83( " ) | 6.02 "<br>6.44 " | 6.60~7.60(m,3H)<br>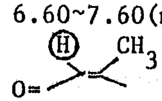 6.14(S,1H)<br>-CH$_3$ 2.39(S,3H) | 1720<br>1700sh | 1620<br>1605 | white (76~80)<br>Bz-hex |
| 4-26 | 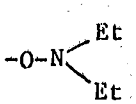 | 3.76 " | 4.44(S,2H) | 5.84(S,1H)<br>6.28 " | 1.13(t,6H)<br>2.74(q,4H) | 1720 | 1630 | colorless oily product |

0117412

Table 4 (cont'd)

Compound II (B=COOCH$_3$ in formula (I))

| Compound No. | A(=-T-D) | NMR ($CDCl_3$) δ value: $-CO_2CH_3$ | $-T-CH_2-$ | $=CH_2$ | T-portion | IR ($cm^{-1}$): $\nu_{c=o}$ | $\nu_{c=c}$ | Property (melting point) |
|---|---|---|---|---|---|---|---|---|
| 4-27 | 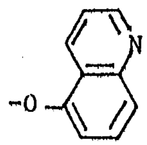 | 3.80(S,3H) | 4.91(S,2H) | 6.06(S,1H)<br>6.43 " | 6.86(d,1H)<br>7.10~7.80(m,3H)<br>8.54(d,1H)<br>8,88(d,1H) | 1705 | 1618 | light yellow crystal<br>(74~76)<br>hex |
| 4-28 | 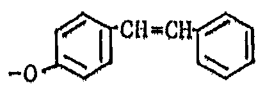 | 3.78( " ) | 4.76( " ) | 6.00 "<br>6.38 " | 6.70~7.80(m,11H) | 1730 | 1635 | white<br>(112~121)<br>Bz-hex |

Table 5

Compound II (A=YQ-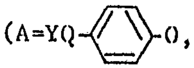-O, B=$COOCH_3$ in formula (I))

| Compound No. | YQ- | NMR ($CDCl_3$) δ value: $-COOCH_3$ | NMR ($CDCl_3$) δ value: 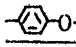-O-$CH_2$- | NMR ($CDCl_3$) δ value: $=CH_2$ | NMR ($CDCl_3$) δ value: YQ moiety | IR ($cm^{-1}$): $\nu_{c=o}$ | IR ($cm^{-1}$): $\nu_{c=c}$ | Property (melting point) |
|---|---|---|---|---|---|---|---|---|
| 5-1 | 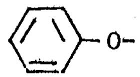 | 3.77(s,3H) | 4.72(s,2H) | 6.02(s,1H)<br>6.39(s,1H) | 6.60∿7.40(m,9H) | 1715$cm^{-1}$ | 1632$cm^{-1}$ | yellow oily product |
| 5-2 | 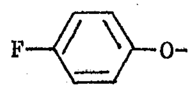 | 3.78( " ) | 4.73( " ) | 6.00( " )<br>6.39( " ) | 6.70∿7.10(m,8H) | 1720 | 1632 | yellow oily product |
| 5-3 | 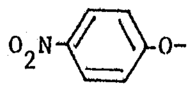 | 3.83( " ) | 4.77( " ) | 6.03( " )<br>6.44( " ) | 6.90∿7.10(m,4H)<br>6.96 (d 2H)<br>8.16 (d 2H) | 1720 | 1635 | light yellow oily product |
| 5-4 | 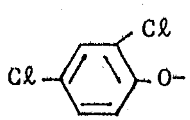 | 3.78( " ) | 4.72( " ) | 5.98( " )<br>6.38( " ) | 6.70∿7.40(m,7H) | 1710 | 1630 | yellow oily product |
| 5-5 | 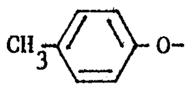 | 3.76( " ) | 4.69( " ) | 5.97( " )<br>6.35( " ) | 6.70∿7.40(m,8H)<br>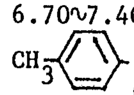 2.28 (s,3H) | 1715 | 1635 | light yellow oily product |
| 5-6 | 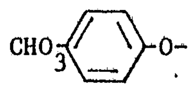 | 3.77( " ) | 4.69( " ) | 5.97( " )<br>6.35( " ) | 6.60∿7.00(m,8H)<br>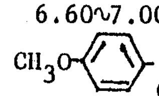 3.74 (s,3H) | 1720 | 1628 | white (68∿69.5°C) |
| 5-7 | 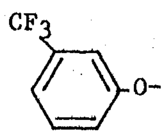 | 3.80( " ) | 4.74( " ) | 6.02( " )<br>6.42( " ) | 6.90∿7.50(m,8H) | 1710 | 1630 | yellow oily product |

Table 5 (cont'd)

Compound II (A=YQ-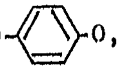-O, B=$COOCH_3$ in formula (I))

| Compound No. | YQ- | NMR ($CDCl_3$) δ value: $-COOCH_3$ | NMR ($CDCl_3$) δ value: 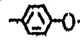-O-$CH_2$- | NMR ($CDCl_3$) δ value: $=CH_2$ | NMR ($CDCl_3$) δ value: YQ moiety | IR ($cm^{-1}$): $\nu_{c=o}$ | IR ($cm^{-1}$): $\nu_{c=c}$ | Property (melting point) |
|---|---|---|---|---|---|---|---|---|
| 5-8 | 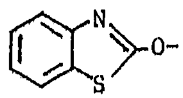 | 3.80(s,3H) | 4.67(s,2H) | 6.00(s,1H)<br>6.40( " ) | 6.60∿6.80(m,4H)<br>6.90∿7.50(m,4H) | 1720 | 1635 | low melting point white solid |
| 5-9 | 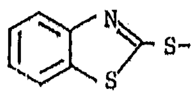 | 3.80( " ) | 4.80( " ) | 6.02( " )<br>6.43( " ) | 6.70∿7.90(m,8H) | 1720 | 1640 | light yellow oily product |
| 5-10 | 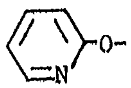 | 3.80( " ) | 4.78( " ) | 6.05( " )<br>6.44( " ) | 6.84∿7.24(m,6H)<br>7.68 (m,1H)<br>8.20 (m,1H) | 1710 | 1635 | yellow (72∿72.5°C) |
| 5-11 | 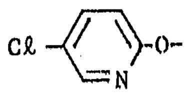 | 3.76( " ) | 4.72( " ) | 5.98( " )<br>6.36( " ) | 6.76(d,1H)6.96(m,4H)<br>7.56(d,d1H)8.04(d,1H) | 1710 | 1635 | colorless oily product |
| 5-12 | 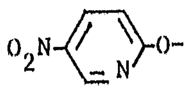 | 3.78( " ) | 4.74( " ) | 6.02( " )<br>6.40( " ) | 6.96(d,1H)7.02(m,4H)<br>8.40(dd,1H)9.00(d,1H) | 1705 | 1635 | yellow (78∿79.5°C) |
| 5-13 | 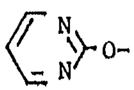 | 3.78( " ) | 4.74( " ) | 6.00( " )<br>6.38( " ) | 6.86∿7.14(m,5H)<br>8.50 (d,2H) | 1715 | 1630 | white (103∿105°C) |
| 5-14 | 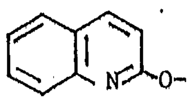 | 3.80( " ) | 4.78( " ) | 6.02( " )<br>6.40( " ) | 6.86∿8.10(m,10H) | 1710 | 1630 | white (87∿88.5°C) |
| 5-15 | 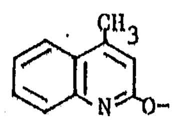 | 3.78( " ) | 4.74( " ) | 6.02( " )<br>6.38( " ) | 7.84∿7.90(m,9H)<br>$-CH_3$ 2.62(s,3H) | 1705 | 1630 | white (97∿100°C) |

0117412

Table 5 (cont'd)

Compound II (A=YQ-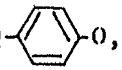-O, B=$COOCH_3$ in formula (I))

| Compound No. | YQ- | NMR ($CDCl_3$) δ value | | | | IR ($cm^{-1}$) | | Property (melting point) |
|---|---|---|---|---|---|---|---|---|
| | | $-COOCH_3$ | 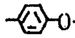-O-$CH_2$- | $=CH_2$ | YQ moiety | $\nu_{c=o}$ | $\nu_{c=c}$ | |
| 5-16 | 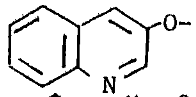 | 3.80(s,3H) | 4.76(s,2H) | 6.03(s,1H)<br>6.42( " ) | 6.80∿8.80(m,6H) | 1705 | 1630 | white<br>(70∿73°C) |
| 5-17 | 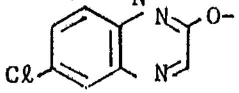 | 3.81( " ) | 4.77( " ) | 6.04( " )<br>6.43( " ) | 6.70∿7.30(m,4H)<br>7.30∿8.70(m,4H) | 1715 | 1635 | white<br>(90∿94°C) |
| 5-18 | 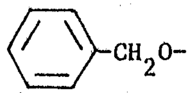 | 3.77( " ) | 4.70( " ) | 5.97( " )<br>6.36( " ) | 6.87 (m,4H)<br>7.10∿7.60(m,5H) | 1720 | 1635 | white<br>(68∿71°C) |
| 5-19 | 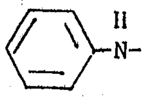 | 3.77( " ) | 4.72( " ) | 6.02( " )<br>6.38( " ) | 6.70∿7.40(m,9H)<br>-N(H)- 5.53(bs 1H) | 1710 | 1632 | brown oily<br>product |
| 5-20 | 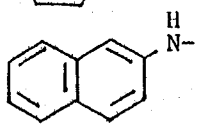 | 3.80( " ) | 4.77( " ) | 6.04( " )<br>6.42( " ) | 6.60∿7.90(m,11H)<br>-N(H)- 5.63(bs 1H) | 1705,1690 | 1622 | light yellow<br>(105∿107°C) |
| 5-21 | 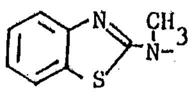 | 3.78( " ) | 4.78( " ) | 6.00( " )<br>6.39( " ) | 6.90∿7.70(m,8H)<br>N-$CH_3$ 3.57(s,3H) | 1730 | 1635 | white<br>(82∿85°C) |

Table 6

Compound III (A=D-T-, B=CN in formula (I))

| Compound No. | D | T | NMR($CDCl_3$) δ value | IR ($cm^{-1}$) | Property |
|---|---|---|---|---|---|
| 6-1 | phenyl | O | 4.60(2H), 6.10(2H), 6.80~7.20(5H) | 2240, 1620 | colorless oily product |
| 6-2 | 4-methylphenyl | O | 2.16(3H), 4.56(2H), 6.08(2H), 6.78(2H), 7.08(2H) | 2240, 1620 | colorless oily product |
| 6-3 | 4-chlorophenyl | O | 4.58(2H), 6.11(2H), 6.82(2H), 7.24(2H) | 2230, 1625 | yellow oily product |
| 6-4 | 4-methoxyphenyl | O | 3.76(3H), 4.57(2H), 6.11(2H), 6.87(4H) | 2230, 1630 | yellow oily product |
| 6-5 | 3,4-dimethylphenyl | O | 2.20(3H), 2.24(3H), 4.60(2H), 6.14(2H), 6.67~7.20(3H) | 2240, 1614 | colorless oily product |
| 6-6 | 4-isopropylphenyl | O | 1.20(6H), 2.86(1H), 4.58(2H), 6.10(2H), 6.77~7.20(4H) | 2240, 1635 | yellow oily product |
| 6-7 | 3,4-dichlorophenyl | O | 4.60(2H), 6.12(2H), 6.77(1H), 7.00(1H), 7.34(1H) | 2240, 1630 | light yellow crystal (mp 35~37°C) |
| 6-8 | phenyl | S | 3.64(2H), 5.68(1H), 5.84(1H), 7.20~7.60(5H) | 2240, 1615 | yellow oily product |

Table 6 (cont'd)

Compound III (A=D-T-, B=CN in formula (I))

| Compound No. | D | T | NMR($CDCl_3$) δ value | IR ($cm^{-1}$) | Property |
|---|---|---|---|---|---|
| 6-9 | 4-methylphenyl | S | 2.30(3H), 3.52(2H), 5.52(1H), 5.72(1H), 7.10(2H), 7.28(2H) | 2230, 1615 | colorless oily product |
| 6-10 | 2-methylphenyl | S | 2.11, 2.23(3H), 3.55(2H), 5.55(1H), 5.74(1H), 7.00∿7.30(4H) | 2250, 1620 | yellow oily product |
| 6-11 | 4-methoxyphenyl | S | 3.48(3H), 3.78(2H), 5.46(1H), 5.74(1H), 6.85(2H), 7.40(2H) | 2220, 1620 | yellow oily product |
| 6-12 | 4-aminophenyl | S | 3.40(2H), 3.70(2H), 5.40(1H), 5.70(1H), 6.56(2H), 7.22(2H) | 2240, 1625 3440, 3470 | brown oily product |
| 6-13 | 4-chlorophenyl | S | 3.56(2H), 5.58(1H), 5.80(1H), 7.25(4H) | 2220, 1640 | colorless oily product |
| 6-14 | 3,4-dichlorophenyl | S | 3.62(2H), 5.69(1H), 5.84(1H), 7.16∿7.48(3H) | 2220, 1620 | white crystal (mp 46∿49°C) |
| 6-15 | 2,4,5-trichlorophenyl | S | 3.70(2H), 5.75(1H), 5.91(1H), 7.48(1H), 7.52(1H) | 2220, 1605 | white crystal (mp 97.5∿100.5°C) |
| 6-16 | 1-naphthyl | S | 3.56(2H), 5.22(1H), 5.56(1H), 7.26∿8.50(7H) | 2240, 1625 | yellow oily product |

Table 6 (cont'd)

Compound III (A=D-T-, B=CN in formula (I))

| Compound No. | D | T | NMR($CDCl_3$) δ value | IR ($cm^{-1}$) | Property |
|---|---|---|---|---|---|
| 6-17 | 2-naphthyl | S | 3.60(2H), 5.50(1H), 5.65(1H), 7.25∿7.80(7H) | 2220, 1620 | white crystal (mp 57∿58.5°C) |
| 6-18 | benzyl | S | 3.12(2H), 3.68(2H), 5.72(1H), 5.90 (1H), 7.28(5H) | 2230, 1620 | colorless oily product |
| 6-19 | 4-chlorobenzyl | S | 3.13(2H), 3.65(2H), 5.73(1H), 5.92(1H), 7.25(4H) | 2240, 1625 | colorless oily product |
| 6-20 | 2-pyridyl | S | 4.02(2H), 5.88(1H), 6.02(1H), 6.90∿8.40(4H) | 2230, 1615 | yellow oily product |
| 6-21 | 2-pyrimidyl | S | 4.00(2H), 5.94(1H), 6.12(1H), 7.04(1H), 8.50(2H) | 2225, 1615 | yellow oily product |
| 6-22 | benzothiazolyl-2-yl | S | 4.16(2H), 6.00(1H), 6.14(1H), 7.20∿7.90(4H) | 2230, 1620 | yellow oily product |
| 6-23 | cyclohexyl | S | 1.20∿2.10(10H), 2.64(1H), 3.32(2H), 5.88(1H), 5.94(1H) | 2230, 1620 | yellow oily product |

Table 7

Compound III (A=D-N(R)-, B=CN in formula (I))

| Compound No. | D | R | NMR($CDCl_3$) δ value | IR ($cm^{-1}$) | Property |
|---|---|---|---|---|---|
| 7-1 | phenyl | H | 3.84(2H), 4.04(1H), 5.90(2H), 6.50∿7.24(5H) | 2220, 1615 | yellow oily product |
| 7-2 | 4-methylphenyl | H | 2.26(3H), 3.90(3H), 5.98(2H), 6.48∿7.05(4H) | 2220, 1630 | yellow oily product |
| 7-3 | 2-chlorophenyl | H | 4.00(2H), 4.72(1H), 5.90(1H), 5.96(1H), 6.50∿7.18(4H) | 2240, 1620 | yellow oily product |
| 7-4 | 3-chlorophenyl | H | 3.80(2H), 4.20(1H), 5.84(1H), 5.88(1H), 6.25∿7.04(4H) | 2225, 1620 | yellow oily product |
| 7-5 | 4-chlorophenyl | H | 3.92(2H), 4.12(1H), 5.96(1H), 6.00(1H), 6.47∿7.20(3H) | 2230, 1625 | yellow oily product |
| 7-6 | 3,4-dichlorophenyl | H | 3.90(2H), 4.32(1H), 5.92(1H), 5.98(1H), 6.35∿7.20(3H) | 2225, 1625 | yellow oily product |
| 7-7 | phenyl | meth-yl | 2.92(3H), 3.98(2H), 5.68(1H), 5.88(1H), 6.57∿7.24(5H) | 2230, 1620 | yellow oily product |
| 7-8 | α-methylbenzyl | H | 1.35(3H), 1.64(1H), 3.24(2H), 3.80(1H), 5.98(1H), 6.02(1H), 7.25(5H) | 2230, 1620 | light yellow oily product |
| 7-9 | 2-pyridyl | H | 4.15(2H), 5.04(1H), 5.94(2H), 6.37∿8.08(4H) | 2220, 1620 | brown oily product |

Table 8

Compound IV ($A=C_6H_5O$ in formula (I))

| Compound No. | -B | NMR ($CDCl_3$) δ value: -B | NMR: $-O-CH_2-$ | NMR: $=CH_2$ | NMR: $C_6H_5O-$ | IR($cm^{-1}$): $\nu_{c=o}$ | IR($cm^{-1}$): $\nu_{c=c}$ | Property (melting point) |
|---|---|---|---|---|---|---|---|---|
| 8-1 | $COOC_6H_5$ | *1 | 4.82(s,2H) | 6.15(s,1H)<br>6.58( " ) | 6.70∿7.50(m,10H) among them, 5H is ester portion*1 | 1720 | 1630 | colorless oily product |
| 8-2 | $CONHC_6H_5$ | H<br>-N- 8.32(bs 1H)<br>$N-C_6H_5$ *1 | 4.84( " ) | 5.78( " )<br>6.20( " ) | 6.70∿7.70(m,10H) among them, 5H is amide portion*1 | 1655 | 1617 | white (84.5∿87°C) |
| 8-3 | $CONEt_2$ | $N-CH_2CH_3$ (1.12(t, 6H) 3.41(q, 4H) | 4.74( " ) | 5.24( " )<br>5.47( " ) | 6.70∿7.30(m, 5H) | 1610*2 | *2 | colorless oily product |
| 8-4 | $CON{<}^{CH_3}_{CH_2C_6H_5}$ | $N-CH_3$ 4.67(s, 2H)<br>$N-CH_2-$<br>$-C_6H_5$ *1 | 4.80( " ) | 5.34( " )<br>5.57( " ) | 6.70∿7.40(m,10H) among them 5H is amide portion*1 | 1620*2 | *2 | colorless oily product |
| 8-5 | $CONHC_6H_4SO_2NH_2$ (1.4) | H<br>-N- 6.43 (bs, 1H)<br>$-N-C_6H_4-SO_2$ 7.80 8.00 (m, 4H)<br>$SO_2-NH_2$ 2.95 (bs, 2H) | 4.90( " ) | 5.90( " )<br>6.17( " ) | 6.80∿7.40 (m, 5H) | 1660 | 1620 | white (161∿163°C) |
| 8-6 | $COSC_6H_5$ | *1 | 3.78( " ) | 5.69( " )<br>6.24( " ) | 6.70∿7.50(m,10H) among them, 5H is amide portion*1 | 1730<br>1670 | 1620 | colorless oily product |
| 8-7 | $COON=C(CH_3)_2$ | $=C(CH_3)_2$ 2.00(s, 3H)<br>2.08( " ) | 4.80( " ) | 6.07( " )<br>6.44( " ) | 6.70∿7.40(M, 5H) | 1730 | 1635 | colorless oily product |

*1: Overlap of signal
*2: Overlap of absorption

Table 9

Compound V (B=COOH in formula (I))

| Compound No. | A | NMR ($CDCl_3$) δ value -COOH | $-X-CH_2-$ | $=CH_2$ | A | IR ($cm^{-1}$) $\nu_{c=o}$ | $\nu_{c=c}$ | Property (mp °C) |
|---|---|---|---|---|---|---|---|---|
| 9-1 | 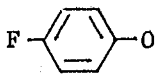 | 10.18 (bs, 1H) | 4.77 (s, 2H) | 6.21(s,1H) 6.64( " ) | 6.80~7.20(m, 4H) | 1680 | 1628 | white (123~129) |
| 9-2 | 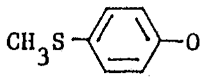 | 10.32 ( " ) | 4.73 ( " ) | 6.12( " ) 6.54( " ) | 6.70~7.40(m, 4H) $-SCH_3$ 2.43(s,3H) | 1680 1715 | 1627 | white (88~92) hex |
| 9-3 | 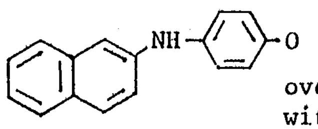 | overlap with the signal of naphthyl | 4.75 ( " ) | 6.03( " ) 6.43( " ) | 6.60~7.90(m,11H) among them, 1H is carboxyl | 1695 | 1630 | grey (145~148) |
| 9-4 | 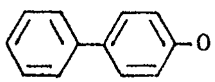 | overlap with the signal of biphenyl | 4.82 ( " ) | 6.07( " ) 6.41( " ) | 6.80~8.00(m,10H) among them, 1H is carboxyl | 1708 | 1635 | white (157~163) EtOH |
| 9-5 | 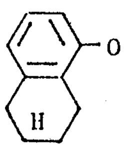 | 9.10 (bs, 1H) | 4.75 ( " ) | 6.17( " ) 6.55( " ) | 1.50~2.00(m, 4H) 2.40~2.90(m, 4H) 6.50~7.30(m, 3H) | 1685 | 1630 | white (152~154) Bz-hex |

Table 10

Compound VI ( 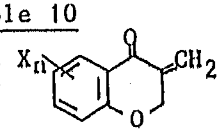 , i.e., 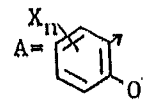 , B=CO in formula (I))

| Compound No. | $X_n$ | NMR ($CDCl_3$) δ value | IR ($cm^{-1}$) | Property |
|---|---|---|---|---|
| 10-1 | 6-methyl | 2.25(3H), 4.90(2H), 5.49(1H), 6.22(1H), 6.70∿7.80(3H) | 1683, 1620 | light yellow oily product |
| 10-2 | 6-chloro | 4.98(2H), 5.57(1H), 6.29(1H) 6.90, 7.39, 7.88 (1H each) | 1680, 1625 | light yellow oily product |
| 10-3 | 6-methoxy | 3.78(3H), 4.93(2H), 5.55(1H), 6.27(1H), 6.80∿7.50(3H) | 1680, 1620 | yellow oily product |
| 10-4 | 6-fluoro | 4.97(2H), 5.57(1H), 6.30(1H), 6.70∿7.70(3H) | 1687, 1620 | white crystal (mp 168∿172°C) |
| 10-5 | 5-chloro | 5.01(2H), 5.58(1H), 6.30(1H), 6.80∿7.20(2H), 7.90(1H) | 1682, 1622 | white crystal (mp 88∿102°C) |
| 10-6 | 7-methyl | 2.32(3H), 4.78(2H), 6.28(1H), 6.53(1H), 6.60∿7.40(3H) | 1680, 1620 | yellow oily product |
| 10-7 | 6,8-dichloro | 5.10(2H), 5.67(1H), 6.36(1H), 7.55(1H), 7.83(1H) | 1682, 1623 | white crystal (mp 87∿90°C) |
| 10-8 | 6,7-dichloro | 5.00(2H), 5.60(1H), 6.52(1H), 7.10(1H), 7.97(1H) | 1676, 1623 | white crystal (mp 75∿85°C) |

sh: observed as shoulder

The compounds of the present invention can be easily synthesized by the following processes. The reaction conditions (e.g., temperature, time, molar ratio, solvent, etc.) and isolation and purification means can be defined by the conventional techniques.

Compound II (B = COOR in formula (I)):

[Synthesis - 1]

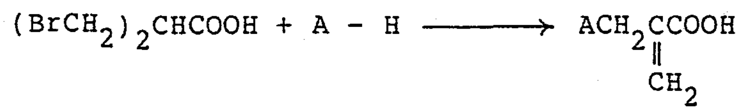

(2) (1) (3)

Compound (3) is equal to Compound (V).

[Synthesis - 2]

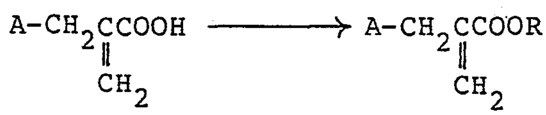

(3) (4)

Compound (4) is equal to Compound (II).

[Synthesis - 3]

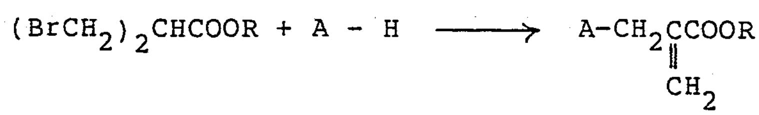

(5) (1) (4)

[Synthesis - 4]

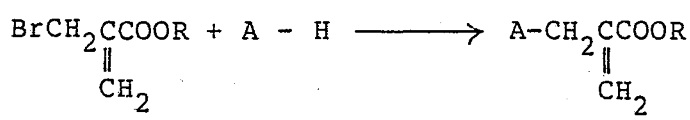

(6) (1) (4)

Synthesis-1 is a process for obtaining α-methylene-

propionic acids (Compound (3)) substituted with A at the β-position thereof by reacting phenol, thiophenol, etc., represented by A-H (Compound (1)) (wherein A is the same as in Table 3 with β,β'-dibromoisobutyric acid (Compound (2)) in the presence of a solvent such as ethanol, etc., and an alkali (for example, 3-fold moles of NaOH per Compound (2)). Further, Compound (3) is esterified to obtain the desired β-substituted-α-methylenepropionic acid esters (Compound (4)) which is Synthesis-2.

In Synthesis-3, a phenol, thiophenol, etc., i.e., Compound (1), similar to those described above, is reacted with a β,β'-dibromobutyric acid ester (Compound (5) in an inert solvent such as diethyl ether or tetrahydrofuran (THF) in the presence of a base (2-fold moles per Compound (5)) such as triethylamine to obtain product (4).

Synthesis-4 is a process for obtaining product (4) by reacting a similar phenol, thiophenol, etc., i.e., Compound (1), with an α-bromomethylacrylic acid ester (Compound (6)) in an inert solvent, e.g., diethyl ether, THF, etc. in the presence of a base (equimolar amount to that of Compound (6)) such as triethylamine.

In the above equations, R represents a methyl group; in the case of other lower alkyl groups such as ethyl, propyl, butyl, etc., synthesis can be performed in a similar fashion.

Compound III (B = CN in formula (I)):

[Synthesis - 5]

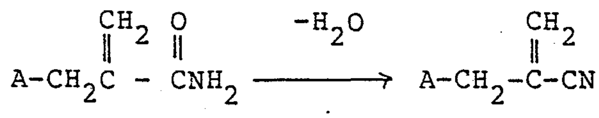


(7) (III)

Substituted acrylamides represented by general formula (7) (wherein A is the same as described for general formula (III) in Table 3) are reacted with dehydrating agents such as thionyl chloride, if necessary in the presence of an inert solvent. The reaction can be carried out at reaction temperatures from room temperature to the boiling point of the inert solvent used.

[Synthesis - 6]

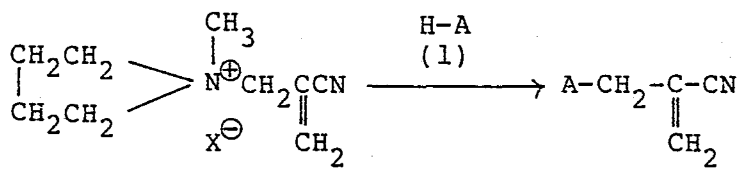


(8) (III)

Pyrrolidinium compounds represented by general formula (8) (wherein X represents a halogen atom) are reacted with a hydroxyl compound, mercapto compound or amino compound represented by general formula (1) (where A is the same as described for general formula (III) above) in an inert solvent. The compounds represented by general formula (8) can be obtained by quaternizing 2-(1-pyrrolidinyl)methylacrylonitrile (Compound (9)) with an alkyl halide in a conventional manner. As inert solvents, conventional organic solvents such as diethyl ether, THF, dimethylformamide, dimethyl sulfoxide, acetonitrile, etc. are employed. The reaction is carried out at a reaction temperature of room temperature to the boiling point of a solvent used, preferably room temperature or under mild heating condition.

Starting material (9) is easily obtained from cyanoacetic acid, formalin and pyrrolidine in accordance with the known process described in Adrian, G. Bull. Soc. Chim. Fr., 1971, (11), 4160 - 4169.

Compound IV (B = COZ in formula (I)):

Amines, phenols, thiophenols, etc., represented by formula HZ (wherein Z is an atomic group obtained by removing CO from B at Column IV in Table 2) are reacted with 2-phenoxymethylacrylic acid (Compound (11)) in an inert solvent in the presence of a dehydration-condensing agent (Synthesis-7); alternatively, HZ (10) is reacted with an α-phenoxymethylacrylic halide (Compound (12)) in an inert solvent in the presence of a base in a similar fashion (Synthesis-8).

As inert solvents used in these reactions, halogen containing solvents such as methylene chloride, chloroform, etc.; ether solvents such as diethyl ether, THF, etc.; hydrocarbon solvents such as benzene, toluene, etc.; or ethyl acetate and the like are appropriate. As dehydration-condensing agents used for condensation, compounds which are conventionally used as dehydration-condensing agents, such as quaternary pyridinium salts such as 2-chloro-1-methyl-pyridinium tosylate, etc., N,N-dicyclohexylcarbodiimide (DCC), ethyl chloroformate, etc. can be employed. Among the condensing agents exemplified above, the presence of a base is required in the case of using other than DCC. As bases, as long as they have poor nucleophilicity, any base(s) selected from tertiary amines, e.g., triethylamine; pyridines; anilines; and the like may be used. The dehydration-condensing agent and base can be used in stoichiometric amounts to Compound (12) in accordance with the conventional manners. The reaction temperatures can be in the range of -30°C to 100°C, preferably -10°C to 30°C.

2-Phenoxymethylacrylic acid halides used in Synthesis-8 are, for example, 2-phenoxymethylacrylic acid chloride, which can easily be synthesized from 2-phenoxymethylacrylic acid in a conventional manner.

These syntheses are illustratively shown below.

[Synthesis - 7]

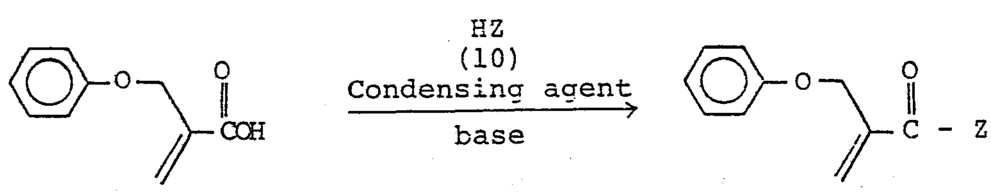


(11) (IV)

[Synthesis - 8]

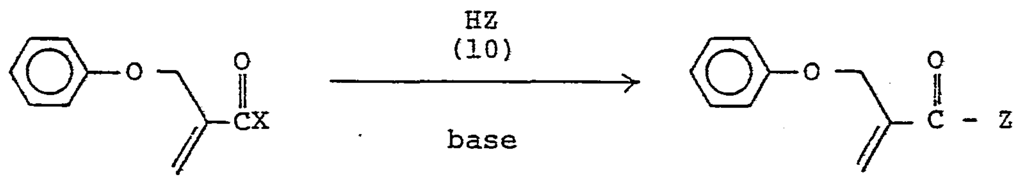


(12)

(IV)

(X = halogen)

Compound V (B = COOH in formula (I))

This compound can be synthesized in the same manner as has been described for intermediate (3) of Compound I in Synthesis-1, wherein A represents an atomic group described at Column V in Table 3.

Compound VI (B = CO in formula (I))

[Synthesis - 9]

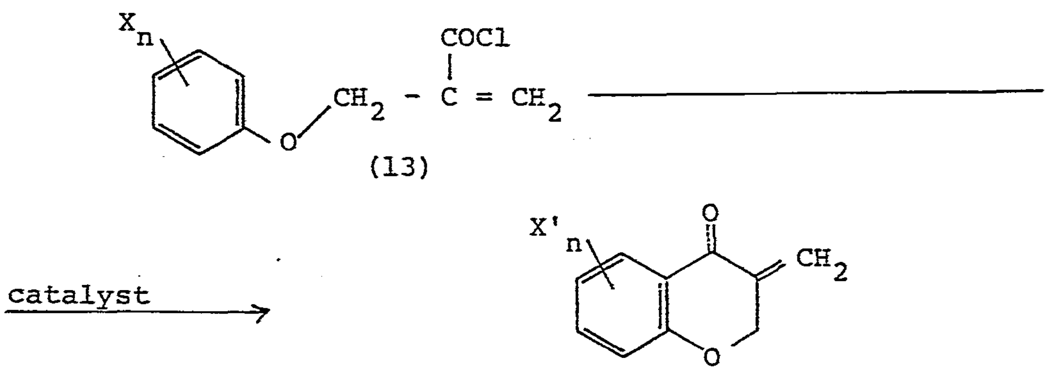


(13)

(VI)

(X' = methyl, methoxy, chloro or fluoro; n = 1 or 2)

Synthesis-9 is a process which comprises subjecting an acid chloride (Compound (13)) easily obtained from the corresponding carboxylic acid in a conventional manner to intramolecular cyclization at temperatures of 0 to 30°C in an inert solvent in the presence of a small excess (1.01 to 1.10-fold moles per Compound (13)) of a catalyst (e.g., aluminum chloride, zinc chloride, etc.; a Friedel-Crafts catalyst) to obtain the desired substituted-3-methylene-4-chromanone I. In the reaction equation, X has the same meaning as at column VI in Table 3.

More detailed examples of syntheses are shown by preparation examples and representative physical values of the products obtained shown in Tables 4 to 10.

Actions and Effects of the Invention:

The actions of the compounds of the present invention on plants are shown in the test examples.

Unlike a pharmacological activity such as a blood pressure depressants, etc., and an action on a plant such as growth acceleration, acceleration of falling leaves, etc., possessed by known α-substituted acrylic acid derivatives, the compounds of the present invention exhibit a growth inhibiting action on plants.

Specific synthesis examples are now given (Preparation Examples). Unless otherwise indicated, all steps were carried out at room temperature and at atmospheric pressure.

Preparation Example 1 (Synthesis - 1)

Synthesis of 2-methylene-3-(4-fluorophenoxy)propionic acid:

In 20 ml of absolute ethanol was dissolved 1.35 g of 4-fluorophenol (12 mmols). Next, sodium hydroxide powder (0.48 g, 12 mmols) was added to the solution and the mixture heated under reflux for 30 minutes. After it was confirmed that the reaction system became a homogeneous solution, an absolute ethanol solution (10 ml) of 0.98 g of β,β'-dibromoisobutyric acid (4 mmols) was added dropwise to the solution and the reaction was carried out for 4 hours under reflux. After cooling to room temperature, ethanol was removed by distillation under reduced pressure and water was added to the residue. The mixture was rendered acidic (pH 2 to 3) with 10% hydrochloric acid and extraction was performed with diethyl ether. The ethereal layer was extracted with aqueous saturated sodium hydrogencarbonate and the alkali-extracted layer again rendered acidic with conc. hydrochloric acid to precipitate white crystals. The precipitated crystals were separated by suction filtration. After washing with water, the crystals were dried under reduced pressure. The resulting crude product was purified by thin layer chromatography (Wako gel$^R$ B-5F; developed with hexane:ethyl acetate (hereafter referred to as H:E) = 7:3 by volume (hereinafter the same) to obtain 0.21 g (yield 53%) of 2-methylene-3-(4-fluorophenoxy)propionic acid (Compound No. 9-1).

Preparation Example 2 (Synthesis - 2)

Synthesis of methyl 2-methylene-3-(4-fluorophenoxy)propionate:

In 20 ml of absolute methanol was dissolved 0.35 g (1.8 mmols) of 2-methylene-3-(4-fluorophenoxy)propionic acid obtained in Preparation Example 1. A catalytic quantity (two drops) of conc. sulfuric acid was added to the solution and the mixture heated under reflux. After reaction for 6 hours, the solvent was removed by distillation under reduced pressure. Water was added to the residue followed by extraction with diethyl ether. After washing with water and with a saturated NaCl solution, the ethereal layer was dried over anhydrous magnesium sulfate. The drying agent was separated by filtration and the ether was removed by distillation under reduced pressure to obtain a crude product. The crude product was purified by thin layer chromatography (the same conditions were used as in Preparation Example 1) to obtain 0.24 g (63%) of methyl 2-methylene-3-(4-fluorophenoxy)propionate (Compound No. 4-15).

In a manner similar to Preparation Examples 1 and 2, Compound Nos. 4-9, 4-11, 4-12, 4-13, 4-17, 4-19, 4-22, 5-8, 5-10, 5-11 and 5-20 were obtained.

Preparation Example 3 (Synthesis - 3)

Synthesis of methyl 2-methylene-3-(4-chlorophenylthio)propi-

onate:

In 25 ml of dry diethyl ether was dissolved 1.2 g (4.62 mmols) of methyl β,β'-dibromoisobutyrate. Under ice cooling, 1.3 ml (9.32 mmols) of triethylamine was added dropwise to the solution under argon atmosphere. Stirring was continued at the same temperature for 30 minutes. Then a dry diethyl ether solution (10 ml) of 0.67 g (4.63 mmols) of 4-chlorothiophenol was added dropwise to the mixture. The reaction temperature was elevated to room temperature and stirring was continued for 3 hours and 30 minutes. After completion of the reaction, the formed precipitate was separated by filtration. After washing with an aqueous saturated sodium bicarbonate solution, water and then a saturated NaCl aqueous solution, the precipitate was dried over anhydrous magnesium sulfate. After separating the drying agent by filtration, the ether was removed by distillation under reduced pressure. The thus obtained crude product was purified by silica gel column chromatography (Wako gel$^R$ C-100, H:E = 9:1 as a solvent for development) to obtain 0.24 g (21%) of methyl 2-methylene-3-(4-chlorophenylthio)propionate (Compound No. 4-2).

Compound Nos. 4-3, 4-4, 4-6, 4-7, 4-14, 4-16, 4-18, 4-20, 4-21, 4-23, 4-24 to 4-28, 5-1 to 5-7, 5-9, 5-12 to 5-19 and 5-21 were obtained in a similar fashion.

<u>Preparation Example 4 (Synthesis - 4)</u>

Synthesis of methyl 2-methylene-3-(phenylthio)propionate:

In a 5 ml of dry THF was dissolved 0.183 g (1 mmol) of methyl α-bromomethylacrylate, and 0.121 mg (1.2 mmols) of triethylamine was added dropwise to the solution under argon atmosphere.

Next, a dry THF solution (3 ml) of 0.117 g (1.0 mmol) of thiophenol was added dropwise to the mixture and stirring was continued for 7 hours under ice cooling. After standing at room temperature overnight, the mixture was diluted with diethyl ether (10 ml) and washed (in order) with a saturated sodium bicarbonate aqueous solution, water and a saturated NaCl aqueous solution. After drying over anhydrous magnesium sulfate, the resulting crude product was purified with thin layer chromatography (B-5F, H:E = 10:1 as a solvent for development) to obtain 0.17 g (82%) of methyl 2-methylene-3-(phenylthio)propionate (Compound No. 4-1).

Compound Nos. 4-5 and 4-8 were obtained in a similar fashion.

Preparation Example 5 (Synthesis - 4)

Synthesis of methyl 2-methylene-3-phenoxypropionate:

In 5 ml of dry benzene was suspended 0.06 g (1.2 mmols) of sodium hydride. While stirring at room temperature, a dry benzene solution of 0.104 g (1.1 mmols) of phenol was added dropwise to the suspension under argon atmosphere. After generation of hydrogen ceased, a dry

benzene solution (3 ml) of 0.18 g (1 mmol) of methyl α-bromomethylacrylate was added dropwise and stirring was continued for 6 hours. After standing at room temperature overnight, the mixture was diluted with diethyl ether (10 ml). Insoluble matter was separated by filtration and the filtrate washed with water and then a saturated NaCl aqueous solution followed by drying over anhydrous magnesium sulfate. After separating the drying agent by filtration, the solvent was removed by distillation under reduced pressure to obtain a colorless oily product. The oily product was purified by thin layer chromatography (Wako gel$^R$ B-5F, H:E=8:2 developer) to obtain 0.09 g (47%) of methyl 2-methylene-3-phenoxypropionate (Compound No. 4-10).

Preparation Example 6 (Synthesis - 5)

Synthesis of 2-phenoxymethylacrylonitrile (Compound No. 6-1):

To a benzene solution (4 ml) of 200 mg (1.13 mmols) of 2-phenoxymethylacrylamide conventionally obtained from 2-phenoxymethylacrylic acid was added 0.12 ml (1.65 mmols) of thionyl chloride and the mixture heated to 80°C. Further 0.25 ml (3.42 mmols) of thionyl chloride was added to the mixture 2 hours later. After heating for 2 hours, benzene and excess of thionyl chloride were removed by distillation under reduced pressure to obtain 190 mg of a yellow oily product. The crude product was purified by thin layer

chromatography (Wako gel[R] B-5F; H:E = 3:1, as a solvent for development) to obtain 90 mg (yield 50.1%) of colorless oily 2-phenoxymethylacrylonitrile. The structure was confirmed by spectral data.

Preparation Example 7 (Synthesis - 5)

Synthesis of 2-(4-methylphenoxy)methylacrylonitrile (Compound No. 6-2):

To a benzene solution (10 ml) of 290 mg (1.52 mmols) of 2-(4-methylphenoxy)methylacrylamide conventionally obtained from 2-(4-methylphenoxy)methylacrylic acid was added 0.6 ml (8.23 mmols) of thionyl chloride and the mixture was refluxed for 8 hours and 20 minutes. By removing benzene and an excess of thionyl chloride by distillation under reduced pressure, 0.32 g of a yellow oily product was obtained. It was confirmed by the analysis of spectrum thereof that the crude product contained the desired 2-(4-methylphenoxy)methylacrylonitrile and its hydrogen chloride addition product. Thus, for purpose of eliminating the hydrogen chloride, the following operation was carried out. The yellow oily product was dissolved in 10 ml of diethyl ether and a 5% NaOH solution (10 ml) was added to the solution. The solution mixture was stirred for 5 hours. The resulting organic layer was washed with water and dried over magnesium sulfate. The solvent was removed by distillation under reduced pressure and 0.2 g of the resulting yellow

oily product was purified by thin layer chromatography (Wako gel$^R$ B-5F; H:E = 3:1 as a solvent for development) to obtain 130 mg (yield 47.5%) of colorless oily 2-(4-methylphenoxy)-methylacrylonitrile. Compound Nos. 6-3 and 6-8 were synthesized by dehydration of the corresponding amides in a similar manner.

Preparation Example 8 (Synthesis - 6)

Synthesis of 2-(2-pyrimidylthio)methylacrylonitrile (Compound No. 6-21):

To a dimethyl sulfoxide solution (2 ml) of 200 mg (0.72 mmol) of a quaternary salt conventionally obtained from 2-(1-pyrrolidinyl)methylacrylonitrile and methyl iodide was added dropwise a dimethyl sulfoxide solution (2 ml) of 80 mg (0.72 mmol) of 2-mercaptopyrimidine. After stirring the mixture for 3 hours at room temperature, the reaction mixture was poured into water (10 ml) and the mixture was extracted with diethyl ether (20 ml). After drying the organic layer over magnesium sulfate, the solvent was removed by distillation under reduced pressure. 90 mg of the resulting yellow oily product was purified by thin layer chromatography (Wako gel$^R$ B-5F; H:E = 1:2 as a solvent for development) to obtain 80 mg (yield 59.4%) of yellow oily 2-(2-pyrimidylthio)methylacrylonitrile.

Preparation Example 9 (Synthesis - 6)

Synthesis of 2-(4-methylphenylthio)methylacrylonitrile (Compound No. 6-9):

To a N,N-dimethylformamide solution (3 ml) of 200 mg (0.72 mmol) of a quaternary salt conventionally obtained from 2-(1-pyrrolidinyl)methylacrylonitrile and methyl iodide was added dropwise an N,N-dimethylformamide solution (3 ml) of 89 mg (0.72 mmol) of p-cresol. After stirring the mixture for 15 minutes at room temperature, the reaction mixture was poured into water (10 ml) and extraction was conducted with diethyl ether (20 ml). After drying the resulting organic layer over magnesium sulfate, the solvent was removed by distillation under reduced pressure. The resulting crude product, 130 mg, was purified by thin layer chromatography (Wako gel$^R$ B-5F; H:E = 3:1 as a solvent for development) to obtain 120 mg (yield 88.2%) of colorless oily 2-(4-methylphenylthio)methylacrylonitrile. Compound Nos. 6-10 to 6-20 and 6-22 to 6-23 were synthesized in a similar fashion.

<u>Preparation Example 10</u>

Synthesis of 2-(phenylaminomethyl)acrylonitrile (Compound No. 7-1):

To an N,N-dimethylformamide solution (3 ml) of a quaternary salt II (300 mg) conventionally obtained from 2-(1-pyrrolidinyl)methylacrylonitrile and methyl iodide was added dropwise an N,N-dimethylformamide solution (3 ml) of aniline (100 mg), while stirring. After completion of the dropwise addition, the mixture was reacted at room temperature for 2 hours and 30 minutes and then at 60°C for 4 hours and 45 minutes. The reaction mixture was cooled to room temperature and then poured into water followed by extraction with diethyl ether. After drying the organic layer over magnesium sulfate, the solvent was removed by distillation under reduced pressure to obtain 160 mg of a yellow oily product. The crude product was purified by thin layer chromatography (Wako gel$^R$ B-5F; H:E = 1:3 as a solvent for development) to obtain 130 mg (yield 78.4%) of yellow oily 2-(phenylaminomethyl)acrylonitrile.

Preparation Example 11

Synthesis of 2-(α-methylbenzylamino)methylacrylonitrile (Compound No. 7-8):

To an N,N-dimethylformamide solution (2 ml) of a quaternary salt (Compound (8)) (200 mg) conventionally obtained from 2-(1-pyrrolydinyl)methylacrylonitrile and methyl iodide was added dropwise an N,N-dimethylformamide solution (2 ml) of α-methylbenzylamine (87 mg) while stirring. After completion of the dropwise addition, the mixture was reacted at room temperature for 2 hours and then at 60°C for 4 hours. After the reaction mixture was cooled, the mixture was poured into water followed by extraction with diethyl ether. After drying the organic layer over magnesium sulfate, the solvent was removed by distillation under reduced pressure to obtain 140 mg of a yellow oily product. It was

clear from the nuclear magnetic resonance spectrum thereof that the desired 2-(α-methylbenzylamino)methylacrylonitrile (and a compound which was assumed to be formed by reacting an additional 1 mole of the quaternary salt (Compound (8)) to the desired product) were present in the crude product. By thin layer chromatography (Wako gel$^R$ B-5F; H:E = 1:3 as a solvent for development) isolation and purification were conducted to obtain 35 mg (yield 26.1%) of light yellow oily 2-(α-methylbenzylamino)methylacrylonitrile.

Preparation Example 12

Synthesis of 2-(2-pyridylamino)methylacrylonitrile (Compound No. 7-9):

While stirring an N,N-dimethylformamide solution (2 ml) of a quaternary salt (8) (200 mg) conventionally obtained from 2-(1-pyrrolydinyl)methylacrylonitrile and methyl iodide, an N,N-dimethylformamide solution (2 ml) of 2-aminopyridine (68 mg) was added dropwise thereto. After completion of the dropwise addition, the mixture was reacted at room temperature for 1 hour and 30 minutes and then at 60°C for 4 hours. The reaction mixture was cooled and then poured into water followed by extraction with diethyl ether. After drying the resulting organic layer over magnesium sulfate, the solvent was removed by distillation under reduced pressure to obtain 70 mg of a black brown oily product. The crude product was purified by thin layer chromatography

(Wako gel[R] B-5F; H:E = 1:3 as a solvent for development) to obtain 50 mg (yield 43.7%) of brown yellow 2-(2-pyridylamino)methylacrylonitrile.

Compound Nos. 7-2 to 7-7 were synthesized in a similar fashion.

Preparation Example 13 (Synthesis - 7)

Synthesis of 2-methylene-3-phenoxypropionic anilide:

In 10 ml of dry methylene chloride were dissolved 0.267 g (1.5 mmols) of 2-methylene-3-phenoxypropionic acid and 0.54 g (1.8 mmols) of 2-chloro-1-methylpyridinium tosylate. While stirring, 0.36 g (3.6 mmols) of triethylamine was added to the solution under ice cooling under argon atmosphere.

Next, an anhydrous methylene chloride (5 ml) solution of 0.168 g (1.8 mmols) of aniline was added dropwise to the mixture and stirring was continued under ice cooling for a further 4 1/2 hours. After completion of the reaction, the reaction mixture was diluted with methylene chloride (20 ml) and washed with, in sequence, a sodium bicarbonate aqueous solution, water and a saturated NaCl aqueous solution. After removing the solvent by distillation, the resulting residue was purified by thin layer chromatography (Wako gel[R] B-5F; H:E = 7:3 as a solvent for development) to obtain 0.19 g (50%) of 2-methylene-3-phenoxypropionic anilide (Compound No. 8-2). Compound Nos. 8-1, 8-3, 8-5 and 8-6 were obtained

in a similar fashion.

Preparation Example 14

Synthesis of O-(2-methylene-3-phenoxypropionyl)acetone oxime:

A solution of anhydrous methylene chloride (10 ml) of 0.33 g of (1.6 mmols) of N,N-dicyclohexylcarbodiimide was added dropwise to a mixture of 0.267 g (1.5 mmols) of 2-phenoxydimethylacrylic acid and 0.12 g (1.6 mmols) of acetone oxime under argon atmosphere while stirring. After stirring was continued at room temperature for 3 1/2 hours, the mixture was diluted with diethyl ether (15 ml) and the precipitate was separated by filtration. The solvent was removed by distillation under reduced pressure and the resulting crude product was purified by thin layer chromatography (the same condition as in Preparation Example 13) to obtain 0.15 g (43%) of O-(2-methylene-3-phenoxypropionyl)-acetone oxime (Compound No. 8-7).

Preparation Example 15 (Synthesis - 8)

Synthesis of N-benzyl-N-methyl-3-phenoxy-2-methylenepropionic amide:

0.182 g (1.5 mmols) of N-benzyl-N-methylamine was dissolved in 10 ml of dry THF. While stirring, 0.182 g (1.8 mmols) of triethylamine was added dropwise to the solution at room temperature under argon atmosphere.

Next, a dry THF (8 ml) solution of 2-phenoxymethylacrylic acid chloride synthesized from 0.267 g (1.5 mmols) of 2-phenoxymethylacrylic acid was added dropwise to the mixture. Stirring was continued for 1 hour and a half and the mixture was allowed to stand overnight at room temperature. The mixture was diluted with diethyl ether (20 ml) and the precipitate formed was separated by filtration. Then, the precipitate was washed with, in sequence, a sodium bicarbonate aqueous solution, water and a saturated NaCl aqueous solution and then dried over magnesium sulfate. The solvent was removed by distillation under reduced pressure to obtain a light yellow crude product. The crude product was purified by thin layer chromatography (the same conditions as in Preparation Example 13) to obtain 0.25 g (59%) of N-benzyl-N-methyl-3-phenoxy-2-methylenepropionic amide (Compound No. 8-4).

Preparation Example 16 (Synthesis - 9)

Synthesis of 6-methyl-3-methylene-4-chromanone (Compound No. 10-1):

A dry benzene solution (100 ml) of 11.2 g of 2-(4-methylphenoxy)methylacrylic acid chloride prepared from 2-(4-methylphenoxy)methylacrylic acid and thionyl chloride in a conventional manner was gradually added dropwise to a dry benzene solution (200 ml) of 7.4 g of aluminum chloride on an ice bath. After stirring was continued for 1 hour at the same temperature, the reaction solution was

cooled and then gently poured into 200 ml of 6N hydrochloric acid. The organic layer was extracted with diethyl ether and the ethereal layer was washed with, in sequence, water, a sodium bicarbonate aqueous solution and water followed by drying over magnesium sulfate overnight. Upon removing the solvent by distillation, 9.3 g of a yellow oily product was obtained. A part of the crude product was purified by thin layer chromatography (Wako gel$^R$ B-5F; H:E = 7:3 as a solvent for development) to obtain a light yellow oily product (0.16 g; yield, 17.3%) and the structure was confirmed by such spectral data.

Preparation Example 17 (Synthesis - 9)

Synthesis of 5-chloro-3-methylene-4-chromanone (Compound No. 10-5):

In a manner similar to Preparation Example 1, 5,6 g of yellow crude product was obtained from 2-(3-chlorophenoxy)methylacrylic acid chloride (10.5 g) prepared from 2-(3-chlorophenoxy)methylacrylic acid. A part (0.4 g) of the crude product was purified by thin layer chromatography (Wako gel$^R$ B-5F; H:E = 7:3 as a solvent for development) to obtain white crystalline 5-chloro-3-methylene-4-chromanone (0.11 g; yield, 12.2%). In this case, no isomer in which a chlorine atom was substituted at the 7-position was obtained. Compound Nos. 10-2 to 10-4 and 10-6 to 10-8 listed in Table 10 were synthesized in a similar fashion.

The desired effect of plant growth inhibition can be achieved when the compounds of the present invention are used in an amount of about 5 to 1,000 g per are.

Test Example (Test of Plant Growth Inhibiting Action)

50 parts of talc, 25 parts of bentonite, 2 parts of Sorpol-9047 (surface active agent manufactured by Toho Chemical Co., Ltd.) and 3 parts of Sorpol-5039 (same as above) were thoroughly mixed to form a carrier. 50 weight parts of test compound was mixed with 200 weight parts of the carrier to prepare a 20% wettable powder. The wettable powder was dispersed in pure water to adjust the amount thereof to a definite concentration (see Table 11 to 17). Seeds of Oryza sativa or rice plant, Echinochloa crus-galli (a kind of millet) and Raphanus sativus (a kind of radish) all of which were forcedly sprouted in a separate dish, were put in the dispersion of wettable powder and grown for 7 days in a lighted thermostat at 25°C and the degree of growth was visually evaluated in accordance with the standard set forth below. The results are shown in Tables 11 to 17.

Standard of Evaluation in Growth Inhibition

| | |
|---|---|
| no influence: | 1 |
| about 25% growth inhibition: | 2 |
| about 50% growth inhibition: | 3 |
| about 75% growth inhibition: | 4 |

about 100% growth inhibition: 5

As shown in Tables 11 to 17, the compounds of the present invention markedly prevented the plants from growth and are expected for application as herbicides.

Table 11

| Compound | Concen-tration (ppm) | Seed of Plant Oryza sativa | Echinochloa crus-galli | Raphanus sativus |
|---|---|---|---|---|
| 4-1 | 100 | 5 | 5 | 2 |
| | 20 | 5 | 5 | 1 |
| 4-2 | 100 | 5 | 5 | 2 |
| | 20 | 5 | 5 | 1 |
| 4-3 | 100 | 5 | 5 | 1 |
| | 20 | 4 | 5 | 1 |
| 4-4 | 100 | 5 | 5 | 2 |
| | 20 | 5 | 5 | 1 |
| 4-5 | 100 | 5 | 5 | 1 |
| | 20 | 4 | 5 | 1 |
| 4-6 | 100 | 5 | 5 | 2 |
| | 20 | 5 | 5 | 1 |
| 4-7 | 100 | 5 | 5 | 1 |
| | 20 | 1 | 4 | 1 |
| 4-8 | 100 | 3 | 2 | 1 |
| | 20 | 1 | 1 | 1 |
| 4-9 | 100 | 5 | 5 | 3 |
| | 20 | 5 | 5 | 1 |
| 4-10 | 100 | 5 | 5 | 5 |
| | 20 | 4 | 5 | 1 |
| 4-11 | 100 | 5 | 5 | 2 |
| | 20 | 4 | 5 | 1 |
| 4-12 | 100 | 5 | 5 | 1 |
| | 20 | 4 | 5 | 1 |
| 4-13 | 100 | 1 | 2 | 1 |
| | 20 | 1 | 2 | 1 |
| 4-14 | 100 | 5 | 5 | 4 |
| | 20 | 5 | 5 | 2 |

Table 11 (cont'd)

| Compound | Concentration (ppm) | Seed of Plant: Oryza sativa | Echinochloa crus-galli | Raphanus sativus |
|---|---|---|---|---|
| 4-15 | 100 | 5 | 5 | 3 |
| | 20 | 3 | 4 | 1 |
| 4-16 | 100 | 5 | 5 | 2 |
| | 20 | 4 | 5 | 1 |
| 4-17 | 100 | 5 | 5 | 1 |
| | 20 | 4 | 5 | 1 |
| 4-18 | 100 | 5 | 5 | 5 |
| | 20 | 4 | 5 | 2 |
| 4-19 | 100 | 5 | 5 | 3 |
| | 20 | 5 | 5 | 1 |
| 4-20 | 100 | 5 | 5 | 1 |
| | 20 | 5 | 5 | 1 |
| 4-21 | 100 | 5 | 5 | 2 |
| | 20 | 1 | 1 | 2 |
| 4-22 | 100 | 5 | 5 | 5 |
| | 20 | 5 | 5 | 3 |
| 4-23 | 100 | 5 | 5 | 2 |
| | 20 | 1 | 1 | 2 |
| 4-24 | 100 | 2 | 2 | 1 |
| | 20 | 1 | 1 | 1 |
| 4-25 | 100 | 5 | 5 | 2 |
| | 20 | 2 | 4 | 1 |
| 4-26 | 100 | 3 | 2 | 1 |
| | 20 | 1 | 1 | 1 |
| 4-27 | 100 | 5 | 5 | 3 |
| | 20 | 4 | 5 | 1 |
| 4-28 | 100 | 2 | 4 | 1 |
| | 20 | 2 | 4 | 1 |

Table 12

| Compound | Concentration (ppm) | Seed of Plant: Oryza sativa | Seed of Plant: Echinochloa crus-galli | Seed of Plant: Raphanus sativus |
|---|---|---|---|---|
| 5-1 | 100 | 5 | 5 | 2 |
| | 20 | 4 | 5 | 1 |
| 5-2 | 100 | 4 | 5 | 1 |
| | 20 | 2 | 4 | 1 |
| 5-3 | 100 | 3 | 3 | 2 |
| | 20 | 1 | 2 | 2 |
| 5-4 | 100 | 1 | 3 | 1 |
| | 20 | 1 | 1 | 1 |
| 5-5 | 100 | 4 | 5 | 5 |
| | 20 | 2 | 4 | 2 |
| 5-6 | 100 | 2 | 4 | 2 |
| | 20 | 1 | 1 | 2 |
| 5-7 | 100 | 3 | 4 | 1 |
| | 20 | 2 | 1 | 1 |
| 5-8 | 100 | 5 | 5 | 5 |
| | 20 | 1 | 4 | 1 |
| 5-9 | 100 | 2 | 5 | 1 |
| | 20 | 1 | 3 | 1 |
| 5-10 | 100 | 5 | 5 | 1 |
| | 20 | 4 | 5 | 1 |
| 5-11 | 100 | 5 | 5 | 1 |
| | 20 | 4 | 5 | 1 |
| 5-12 | 100 | 2 | 4 | 2 |
| | 20 | 2 | 3 | 1 |
| 5-13 | 100 | 5 | 5 | 5 |
| | 20 | 3 | 4 | 1 |
| 5-15 | 100 | 1 | 3 | 1 |
| | 20 | 1 | 3 | 1 |

Table 12 (cont'd)

| Compound | Concen- tration (ppm) | Seed of Plant Oryza sativa | Echinochloa crus-galli | Raphanus sativus |
|---|---|---|---|---|
| 5-16 | 500 | 3 | 2 | 1 |
| | 100 | 2 | 1 | 1 |
| 5-17 | 500 | 3 | 5 | 1 |
| | 100 | 2 | 1 | 1 |
| 5-18 | 100 | 3 | 2 | 1 |
| | 20 | 1 | 1 | 1 |
| 5-19 | 100 | 5 | 5 | 2 |
| | 20 | 4 | 5 | 2 |
| 5-20 | 500 | 2 | 1 | 1 |
| | 100 | 1 | 1 | 1 |

Table 13

| Compound | Concentration (ppm) | Seed of Plant: Oryza sativa | Seed of Plant: Echinochloa crus-galli | Seed of Plant: Raphanus sativus |
|---|---|---|---|---|
| 6-1 | 100 | 5 | 5 | 3 |
| | 20 | 5 | 5 | 1 |
| | 4 | 1 | 5 | 1 |
| 6-2 | 100 | 5 | 5 | 2 |
| | 20 | 5 | 5 | 1 |
| | 4 | 2 | 5 | 1 |
| 6-3 | 100 | 5 | 5 | 5 |
| | 20 | 5 | 5 | 1 |
| | 4 | 2 | 5 | 1 |
| 6-4 | 100 | 5 | 5 | 2 |
| | 20 | 5 | 5 | 2 |
| | 4 | 2 | 4 | 1 |
| 6-5 | 100 | 5 | 5 | 3 |
| | 20 | 5 | 5 | 1 |
| | 4 | 1 | 5 | 1 |
| 6-6 | 100 | 5 | 5 | 1 |
| | 20 | 1 | 5 | 1 |
| 6-7 | 100 | 5 | 5 | 3 |
| | 20 | 5 | 5 | 2 |
| | 4 | 1 | 4 | 1 |
| 6-8 | 100 | 5 | 5 | 3 |
| | 20 | 5 | 5 | 1 |
| 6-9 | 100 | 5 | 5 | 3 |
| | 20 | 3 | 5 | 1 |
| 6-10 | 100 | 5 | 5 | 1 |
| | 20 | 4 | 5 | 1 |

Table 13 (cont'd)

| Compound | Concentration (ppm) | Seed of Plant | | |
|---|---|---|---|---|
| | | Oryza sativa | Echinochloa crus-galli | Raphanus sativus |
| 6-11 | 100 | 5 | 5 | 2 |
| | 20 | 5 | 5 | 2 |
| 6-12 | 100 | 5 | 5 | 2 |
| | 20 | 1 | 3 | 1 |
| 6-13 | 100 | 5 | 5 | 2 |
| | 20 | 5 | 5 | 1 |
| 6-14 | 100 | 5 | 5 | 1 |
| | 20 | 5 | 5 | 1 |
| 6-15 | 100 | 1 | 3 | 1 |
| | 20 | 1 | 1 | 1 |
| 6-16 | 100 | 5 | 5 | 1 |
| | 20 | 5 | 5 | 1 |
| 6-17 | 100 | 3 | 4 | 1 |
| | 20 | 1 | 5 | 1 |
| 6-18 | 100 | 5 | 5 | 5 |
| | 20 | 2 | 5 | 1 |
| 6-19 | 100 | 5 | 5 | 2 |
| | 20 | 2 | 5 | 1 |
| 6-20 | 100 | 5 | 5 | 1 |
| | 20 | 1 | 5 | 1 |
| 6-21 | 100 | 5 | 5 | 2 |
| | 20 | 3 | 4 | 1 |
| 6-22 | 100 | 2 | 5 | 1 |
| | 20 | 1 | 2 | 1 |

Table 14

| Compound | Concentration (ppm) | Seed of Plant: Oryza sativa | Seed of Plant: Echinochloa crus-galli | Seed of Plant: Raphanus sativus |
|---|---|---|---|---|
| 7-1 | 100 | 5 | 5 | 2 |
| | 20 | 1 | 1 | 1 |
| 7-2 | 100 | 5 | 5 | 1 |
| | 20 | 2 | 2 | 1 |
| 7-3 | 100 | 5 | 5 | 1 |
| | 20 | 3 | 4 | 1 |
| 7-4 | 100 | 5 | 5 | 2 |
| | 20 | 2 | 4 | 1 |
| 7-5 | 100 | 5 | 5 | 2 |
| | 20 | 2 | 4 | 1 |
| 7-6 | 100 | 5 | 5 | 2 |
| | 20 | 5 | 5 | 1 |
| 7-7 | 100 | 4 | 5 | 2 |
| | 20 | 1 | 4 | 1 |
| 7-8 | 100 | 5 | 5 | 5 |
| | 20 | 3 | 5 | 1 |
| 7-9 | 100 | 1 | 2 | 1 |
| | 20 | 1 | 1 | 1 |

Table 15

| Compound | Concentration (ppm) | Seed of Plant: Oryza sativa | Echinochloa crus-galli | Raphanus sativus |
|---|---|---|---|---|
| 8-1 | 100 | 5 | 5 | 2 |
| | 20 | 4 | 5 | 1 |
| 8-2 | 100 | 4 | 5 | 1 |
| | 20 | 3 | 4 | 1 |
| 8-3 | 100 | 5 | 4 | 1 |
| | 20 | 2 | 2 | 1 |
| 8-4 | 100 | 5 | 5 | 2 |
| | 20 | 1 | 3 | 1 |
| 8-6 | 100 | 5 | 5 | 2 |
| | 20 | 4 | 5 | 1 |
| 8-7 | 100 | 5 | 5 | 2 |
| | 20 | 4 | 5 | 1 |

Table 16

| Compound | Concentration (ppm) | Seed of Plant: Oryza sativa | Seed of Plant: Echinochloa crus-galli | Seed of Plant: Raphanus sativus |
|---|---|---|---|---|
| 9-1 | 100 | 5 | 5 | 5 |
| | 20 | 5 | 4 | 1 |
| 9-2 | 100 | 5 | 5 | 3 |
| | 20 | 3 | 3 | 1 |
| 9-3 | 100 | 5 | 5 | 2 |
| | 20 | 3 | 2 | 1 |
| 9-4 | 100 | 5 | 5 | 2 |
| | 20 | 5 | 3 | 2 |
| 9-5 | 100 | 5 | 5 | 2 |
| | 20 | 4 | 3 | 2 |

Table 17

| Compound | Concentration (ppm) | Seed of Plant: Oryza sativa | Echinochloa crus-galli | Raphanus sativus |
|---|---|---|---|---|
| 10-1 | 100 | 1 | 3 | 1 |
| | 20 | 1 | 1 | 1 |
| 10-3 | 100 | 5 | 5 | 1 |
| | 20 | 2 | 4 | 1 |
| 10-4 | 100 | 2 | 4 | 1 |
| | 20 | 1 | 1 | 1 |
| 10-5 | 100 | 5 | 5 | 2 |
| | 20 | 1 | 3 | 1 |
| 10-6 | 100 | 4 | 5 | 2 |
| | 20 | 2 | 3 | 1 |
| 10-7 | 100 | 1 | 2 | 1 |
| | 20 | 1 | 1 | 1 |
| 10-8 | 100 | 3 | 5 | 2 |
| | 20 | 2 | 2 | 1 |

While the invention has been described in detail and with reference to specific embodiments thereof, it will be apparent to one skilled in the art that various changes and modifications can be made therein without departing from the spirit and scope thereof.

CLAIMS

1. A propene derivative represented by the general formula:

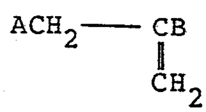

(I)

wherein:

A represents an atomic group bonded to the saturated carbon atom of the propene group via an oxygen atom, a sulfur atom or a nitrogen atom;

B represents an atomic group which is selected from an alkoxy($C_1$-$C_4$)carbonyl group, a cyano group, a substituted aminocarbonyl group represented by the formula, 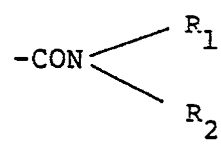 (wherein $R^1$ and $R^2$ represent a group selected from the group consisting of a lower alkyl group having 1 to 4 carbon atoms, phenyl, benzyl and $-C_6H_5SO_2NH_2$), an isopropylideneaminoxycarbonyl group, a phenyloxycarbonyl group, a phenylthiocarbonyl group, a carboxyl group and a carbonyl group which is bound to a benzene nucleus in A to form a 4-chromanone skeleton; and;

A above being the following, depending upon type of Compound (I):

(1) in case that Compound (I) is an alkyl ester or a nitrile:

phenoxy, non-cyclic group-substituted phenoxy (having as substituent(s) one or two selected from the group consisting of methyl, isopropyl, trifluoromethyl, methoxy, methylthio, fluoro, chloro and cyano), phenoxyphenoxy, substituted phenoxyphenoxy (having as substituent(s) one or two selected from the group consisting of fluoro, nitro, chloro, methyl, methoxy and trifluoromethyl), heterocyclic group-substituted oxyphenoxy (having as the substituted oxy group 2-benzothiazolyloxy, 2-pyridyloxy, substituted (chloro- or nitro-)-2-pyridyloxy, 2-pyrimidyloxy, 2-quinolyloxy, 4-methyl-2-quinolyloxy, 3-quinolyloxy or 6-chloro-2-quinoxalyloxy), 2-benzothiazolylthiophenoxy, benzyloxyphenoxy, cyclic aminophenoxy (having as the cyclic amino group anilino, 2-naphthylamino or N-methyl-N-(2-benzothiazolyl)amino), 4-phenylphenoxy, 4-phenylethenylphenoxy, 5-tetralinoxy, 2-fluorenyloxy, 5-quinolyloxy, 4-methyl-7-coumarinyloxy, diethylaminoxy, phenylthio, non-cyclic group-substituted phenylthio (having as substituent(s) one to three groups selected from the group consisting of methyl, methoxy, chloro and amino), benzylthio, 4-chlorobenzylthio, cyclohexylthio, 1-naphthylthio, 2-naphthylthio, 2-pyridylthio, 2-pyrimidylthio, 2-benzothiazolylthio, anilino, substituted anilino (having as the substituent(s) one or two groups

selected from the group consisting of methyl and chloro), N-methylanilino, pyridylamino and α-methylbenzylamino;

(2) in case that Compound (I) is an amide, an oxime ester, a phenyl ester or a thiophenyl ester:

phenoxy;

(3) in case that Compound (I) is a carboxylic acid:

4-fluorophenoxy, 4-methylthiophenoxy, 4-phenylphenoxy, 4-α-naphthylaminophenoxy or 5-tetralinoxy;

(4) in case that Compound (I) is a 4-chromanone compound:

a substituted phenoxy (the substituent(s) being one or two of members selected from the group consisting of methyl, methoxy, chloro and fluoro).

2. The propene derivative of Claim 1 represented by the general formula:

$$\underset{\displaystyle CH_2}{ACH_2\overset{}{\underset{\|}{C}}COOCH_3} \qquad (II)$$

wherein A is as defined in Claim 1.

3. The propene derivative of Claim 1 represented by the general formula:

$$\underset{\displaystyle CH_2}{ACH_2\underset{\|}{C}CN} \qquad (III)$$

wherein A is as defined in Claim 1.

4. The propene derivative of Claim 1 represented by the general formula:

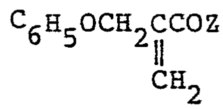

(IV)

wherein Z represents a group selected from the group consisting of $-N<^{R_1}_{R_2}$, $-OC_6H_5$, $-SC_6H_5$ and $-O-N=C(CH_3)_2$, wherein $R^1$ and $R^2$ are as defined in Claim 1.

5. The propene derivative of Claim 1 represented by the general formula:

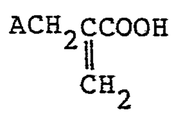

(V)

wherein A is as defined in Claim 1.

6. The propene derivative of Claim 1 represented by the general formula:

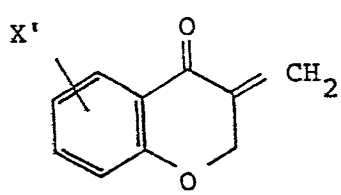

(VI)

wherein X' is selected from a methyl group, a methoxy group, a chlorine atom and a fluorine atom, and n is 1 or 2.

7. The use of the propene derivatives according to any of preceding claims 1 to 5 as plant growth inhibitors.

0117412

European Patent Office

# EUROPEAN SEARCH REPORT

Application number

EP 84100497.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
| --- | --- | --- | --- |
| A | EP - A1 - 0 044 972 (BASF AKTIENGESELLSCHAFT)<br>* Claim *<br>-- | 1,2,4 | C 07 C 59/66<br>C 07 C 69/736<br>C 07 C 101/28<br>C 07 C 101/453<br>C 07 C 103/58<br>C 07 C 101/34<br>C 07 C 101/45<br>C 07 C 143/80<br>C 07 C 149/32<br>C 07 D 213/62<br>C 07 D 239/32<br>C 07 D 241/44<br>C 07 D 311/22<br>A 01 N 37/00<br>A 01 N 39/00<br>A 01 N 43/00 |
| A | DE - A - 1 965 872 (MINNESOTA MINING AND MANUFACTURING CO.)<br>* Examples *<br>-- | 1-3,5 | |
| A | FR - A - 849 822 (I.G. FARBENINDUSTRIE AKTIENGESELLSCHAFT)<br>* Totality *<br>-- | 1,3 | |
| A | DE - B2 - 1 468 344 (MEIJI SEIKA KAISHA LTD.)<br>* Claim 1 *<br>-- | 1 | TECHNICAL FIELDS SEARCHED (Int. Cl. 3) |
| A | GB - A - 1 422 722 (CHINOIN GYOGYSZER ES VEGYESZETI TERMEKEK GYARA, RT.)<br>* Claim 1 *<br>-- | 1 | C 07 C 59/00<br>C 07 C 69/00<br>C 07 C 101/00<br>C 07 C 103/00<br>C 07 C 121/00<br>C 07 C 143/00<br>C 07 C 149/00<br>C 07 D 213/00<br>C 07 D 239/00<br>C 07 D 241/00<br>C 07 D 311/00 |
| A | EP - A1 - 0 060 607 (ICI AUSTRALIA LIMITED)<br>* Abstract *<br>-- | 1,7 | |

The present search report has been drawn up for all claims

| Place of search | Date of completion of the search | Examiner |
| --- | --- | --- |
| VIENNA | 18-04-1984 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0117412

European Patent Office

# EUROPEAN SEARCH REPORT

Application number

EP 84100497.1

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | CHEMICAL ABSTRACTS, vol. 95, no. 10, September 7, 1981, Columbus, Ohio, USA<br>F.E. WARD et al., "Antimicrobial 3-methylene flavanones" pages 110,111 abstract no. 91 568s<br>& J. Med. Chem. 1981, 24(9), 1073-7<br>---- | 1,6 | |
| | | | TECHNICAL FIELDS SEARCHED (Int. Cl.3) |

EPO Form 1503.2 06.78